# EUROPEAN PATENT APPLICATION

(11) **EP 1 719 792 A1**
(43) Date of publication of application: **08.11.2006**
(21) Application number: 05710135.4
(22) Date of filing: 10.02.2005
(51) Int. Cl.: C08G 73/10

(54) **CROSSLINKED POLYIMIDE COMPOUND AND USE THEREOF**

(30) Priority: 13.02.2004 JP 2004036695
(71) Applicant: Mitsui Chemicals, Inc., Minato-ku, Tokyo 105-7117 (JP)
(72) Inventor: KIZUKA, Hisashi, Sodegaura-shi, Chiba 2990265 (JP); KUBOYAMA, Hisaharu, Yokohama-shi, Kanagawa 2470006 (JP); OKUMURA, Kunio, Omuta-shi, Fukuoka 8368610 (JP); KATOH, Toshio, Kawaguchi-shi, Saitama 3330866 (JP)
(74) Representative: Stuart, Ian Alexander
(86) International application number: PCT/JP2005/002083
(87) International publication number: WO 2005/078005

(57) **Abstract**

[Object] To provide a polyimide compound which can be dissolved in a certain solvent and has high molecular weight, and a preparation process thereof which produces the compound by industrially advantageous process.

[Solving Means] A polyimide compound crosslinked with a polyamine which is soluble in the specific solvent is produced by dehydration and condensation of amino acid or salt thereof and polyamine in an organic solvent under the presence of protonic acid. In particular, in the case of using a polyamine having low solubility to an organic solvent such as especially lysine, ornithine, etc., reaction rate of the polymerization can be increased by adding a polyamine in the step before or initial polymerization because water which is contained in the raw material or produced in the dehydration reaction saves as a good solvent for the polyamine.

## Description

### TECHNICAL FIELD

The present invention relates to a polyimide compound which is useful as an intermediate of polyamino acids resin, etc.

### BACKGROUND ART

A polyimide compound is a useful compound as an intermediate for synthesizing a polyamino acid which is used as a base material for cosmetics, or aromatic-cosmetics, etc. For example, since succinimide unit constituting a polyimide compound can be easily reacted with amine compound, polyamino acids having various grafting groups can be synthesized through a compound having succinimide unit as an intermediate. As a raw material for cosmetics and aromatic-cosmetics having excellent thickening property and coating-forming ability, polyamino acid having high molecular weight is preferable, and as the intermediates thereof, polyimide having high molecular weight is desirable.

A process for producing a polyimide compound includes, for example, a process of polymerizing aspartic acid in the presence of condensed phosphoric acid (Patent Document 1) and a process of polymerizing aspartic acid in the organic solvent under the presence of hydrochloric acid (Patent Document 2). However, a long polymerization time is required in order to obtain a polyimide compound having weight average molecular weight of 100,000 or more by these methods. Furthermore, it is difficult to obtain a polyimide compounds having weight average molecular weight of 200,000 or more.

There is a method of copolymerizing a polyimide compound with other monomer to obtain a polyimide compound having higher molecular weight, the method includes, for example, a method of mixing an aspartic acid with lysine and heating (Patent Document 3), and a method of crosslinking a polyimide compound with a hexamethylenediamine in DMF solvent(Patent Document 4). However, polyimide compounds obtained by these methods are gel type and can not be homogeneously dissolved in a solvent. As a raw material for cosmetics or aromatic-cosmetics, a polyamino acid derivative which can be homogeneously dissolved in a solvent is preferable. Therefore, polyimide compound having higher molecular weight which can be homogeneously dissolved in a solvent has been desired.
Patent Document 1 Japanese Laid-Open Publication No. 8-176297
Patent Document 2 Japanese Laid-Open Publication No. 2000-169577
Patent Document 3 US 5,284,936
Patent Document 4 Japanese Laid-Open Publication No. 7-224163

### DISCLOSURE OF THE INVENTION

### PROBLEMS TO BE SOLVED BY THE INVENTION

The object of the present invention is to solve the above problems of prior art and provide polyimide compounds having higher molecular weight which can be produced by industrial advantageous method.

### MEANS FOR SOLVING THE PROBLEMS

In order to solve the problems of the prior art, the present inventors have conducted an extensive investigation. As a result, the inventors have found that polyimide compounds are produced by dehydration and condensation of amino acid or salt thereof and polyamine in the organic solvent under the presence of protonic acid. Thus the present invention has been achieved.

Namely, the present invention relates to the following items (1) to (14):
(1) A polyimide compound crosslinked with polyamine which is soluble in the solvent containing aprotic polar organic solvent.
(2) The polyimide compound accoeding to item (1), wherein the polyimide compound is soluble in said solvent containing aprotic polar organic solvent in a concentration of 5% by mass or more at 25°C.
(3) The polyimide compound according to item (1) or item (2), wherein said polyimide compound is succinimide polymer.
(4) The polyimide compound according to one of items (1) to (3), wherein said aprotic polar organic solvent is the solvent containing a N,N-dimethyl formadmide and/or sulfolane.
(5) The polyimide compound according to one of items (1) to (4), wherein said polyamine is lysine or ornithine.
(6) The polyimide compound according to item (1), wherein the polyimide compound is obtained by dehydration and condensation of amino acid or salt thereof in the presence of polyamine and protonic acid in the solvent containing aprotic polar organic solvent.
(7) The polyimide compound according to item (6), wherein said aprotic polar organic solvent is a solvent containing N,N-dimethylformamide and/or sulfolane.
(8) The polyimide compound according to item (6) or item (7), wherein said protonic acid is hydrochloric acid.
(9) The polyimide compound according to one of items (6) to (8), wherein said polyimide compound is succinimide polymer.
(10) The polyimide compound according to one of items (6) to (9), characterized in that 0.05 to 10 mol% of said polyamine is used with respect to an amount of said amino acid or salt thereof.
(11) The polyimide compound according to one of items (6) to (10), wherein said polyamine is a lysine or an ornithine.
(12) A solution containing a polyimide compound according to any one of items (1) to (11).
(13) A cosmetic containing a polyamino acid polymer derived from polyimide compound according to any one of items (1) to (11).
(14) An external preparation containing a polyamino acid polymer derived from the polyimide compound according to any one of items (1) to (11).

### EFFECT OF THE INVENTION

The high-molecular weight polyimide compound obtained by the preparation process of the present invention is useful as a raw material of polyamino acid derivative having excellent coating-forming ability or thickening property. Further, according to the process of the present invention, polymerization reaction time can be reduced and thus productivity is improved. In this regard, it is industrially advantageous.

### BEST MODE FOR CARRYING OUT THE INVENTION

The present invention is directed to a polyimide compound crosslinked with polyamine which is soluble in the solvent containing aprotic polar organic solvent.

Polyimide compound of the present invention is crosslinked with polyamine. In the present invention, the crosslinking methods include, as suitable examples, but are not limited to, a crosslinking method used in the producing method described hereafter.

For a degree of crosslinking of the polyamine of the present invention, it is preferable that 0.05 to 10% of polyamines are crosslinked with regard to the total imide repeating units of polyimide compounds.

The polyimide compound of the present invention can be dissolved in the solvent containing an aprotic polar organic solvent.

Herein, in general, the aprotic polar organic solvent may be solvent having its property, and includes, but is not limited to, for example, N-methyl-2-pyrrolidone, N,N-dimehtylformamide, N,N-dimethylimidazolidinone, dimethylsulfoxide, sulfolane, and dimethylsulfone. Further, said solvents may be used as a mixture of at least one solvent. Further, with respect to the solubility and the like of the polyimide compound, N,N-dimethylformamide and/or sulfolane is preferable. Especially, sulfolane is preferable.

Further, the solvent containing an aprotic polar organic solvent may be such solvent that sufficiently mix with said aprotic polar organic solvent, and includes, for example, but is not limited to, pentane, hexane, cyclohexane, heptane, octane, nonane, decane, ethylmethylketone, methylisobutylketone, 2-hexanone, 3-hexanene, cyclohexanone, 2-pentanone, benzene, toluene, (mixed) xylene, ethylbenzene, chlorobenzene, dichlorobenzene, etc.

The solubility of the polyimide compound of the present invention may be such solubility that the polyimide compound is soluble in said solvent and is preferably at least 5% by mass at 25°C, but is not limited to. The solubility of the polyimide in the solvent may be generally at least 5% by mass, preferably at least 10% by mass, more preferably at least 20% by mass at 25°C depending on the solvent which is used.

More specifically, the polyimide compound of the present invention preferably includes but is not limited to polysuccinimide polymer.

The molecular weight of the polyimide compounds of the present invention is not specifically limited as long as the product having desired property is obtained. Generally, molecular weight of the polyimide compound is preferably about 2000 to 5000000, more preferably about 10000 to 2000000, and most preferably about 15000 to 1000000. The molecular weight is determined by gel permeation chromatography in N,N-dimethylformamide solution at 45°C on the basis of a polystyrene standard. (If the molecular weight of the polyimide used is lower, the molecular weight of polyamino acid polymer obtained is lower. Further, if the molecular weight of the polyimide used is higher, the molecular weight of the polyamino acid polymer is higher.)

A process of producing the polyimide compound of the present invention, no limitation is specifically imposed, and suitable example is explained with following method.
The polyimide compound of the present invention is obtained by dehydration and condensation of amino acid or salt thereof in the presence of polyamine and protonic acid in the solvent containing aprotic polar organic solvent.

Hereinafter, suitable embodiments for synthesizing a polyimide compound of the present invention will be described.

### [1] Amino Acid

The amino acids used in the present invention specifically include, for example, but are not limited to, aspartic acid, asparagine, glutamic acid, glutamine, etc.
The amino acids may be commonly in L-form, D-form, or mixtures of D-form and L-form. Further, they may be used alone or in a mixture of at least two amino acids.
According to the present invention, preferable example includes aspartic acid.

### [2] Polyamine

The polyamine used in the present invention is not specifically limited. Examples of the polyamines include lysine, ornithine, 1,6-hexanediamine, 1,4-butanediamine, etc. Among them, with respect to safety, lysine, and ornithine are particularly preferable.

In order to obtain a polymer having high molecular weight and to prevent gelling by inhibiting sudden rise of polymer density in the reaction, etc, it is preferable that the amount of the polyamine to be used is preferably 0.05 to 10mol% of polyamine, and more preferably 0.1 to 2mol% of polyamine based on the amino acid.

The timing of addition of polyamine is not specifically limited. However, in case that the polyamine having low solubility to an organic solvent such as lysine, ornithine, etc, is used, it is preferable that polyamine is added before the polymerization or at the initial step of the polymerization, since due to a water presented in the raw material or produced in the dehydration reaction, solubility of polyamine can be kept high and therefore the reaction rate can be increased.

### [3] Protonic acid

According to the present invention, the protonic acids used as a catalyst include, for example, but is not limited to hydrochloric acid, phosphoric acid, methane sulfonic acid, etc. Due to a strong acidity and high volatility, hydrochloric acid is especially preferable.

### [4] Organic Solvent

The organic solvent used in the present invention, but is not limited, may be preferably a mixed solvent of a solvent in which a polyimide compound dissolves and a solvent which functions to remove water presented in the raw material and produced by the reaction out of the system.

The solvents in which the polyimide compound can dissolve include an aprotic polar organic solvent such as N-methyl-2-pyrrolidone, N,N-dimethylformamide, N,N-dimethylimidazolidinone, dimethylsulfoxide, sulfolane, and dimethylsulfone. Further, a mixture of at least one of the above solvents may be used. Due to a solubility of polyimide compound and stability in the presence of acid catalyst at high temperature, N,N-dimethylformaide and/or sulfolane is preferable, and sulfolane is particularly preferable.

A solvent for dehydration is not specifically limited as long as it is azeotropically distilled with the water. Examples include pentane, hexane, cyclohexane, heptane, octane, nonane, decane, ethylmethylketone, methylisobutylketone, 2-hexanone, 3-hexanone, cyclohexanone, 2-pentanone, toluene, (mixed) xylene, ethylbenzene, chlorobenzene, dichlorobenzene, etc.

### [5] Preparation apparatus

The preparation of the polymer of the present invention may be carried out by continuous operation or batch operation.

### [6] Polymerization Concentration

The concentration of polyimide compound in the polymerization reaction is not specifically limited. In terms of rate of polymerization and solubility of polyimide compound, the preferred polymerization concentration is 5 to 80% by weight.

### [7] Polymerization temperature

According to the present invention, a polyimide compound is obtained by polymerization of amino acid (in particular aspartic acid) and polyamine in an organic solvent under the presence of protonic acid catalyst. This polymerization temperature is not specifically limited. However, because of inhibiting a decomposition reaction of amino acid (especially aspartic acid), polyamine, polymer, organic solvent, etc, and high reaction rate, the polymerization temperature is preferably 100 to 230 °C, more preferably 140 to 210°C, most preferably 160 to 180°C.

### [8] Removing Water from a Reaction System.

According to the invention, a molecular weight of polymer is increased by dehydration and condensation reaction. Therefore, in order to proceed the polymerization quickly, it is preferred that water contained in a raw material and water produced from the reaction is removed efficiently. A method for distillating and eliminating water from the reaction system is not specifically limited, and an example includes distillation and elimination by azeotropically distilling of organic solvent with water. In the case of distillation and elimination by azeotropic distillation with water, mixed gases generated from the polymerization system may be returned to the polymerization system after reducing water content by cooling and condensing and then dehydration or separation etc.

### [9] Isolation of Polymer

According to the present invention, the polymer after the reaction is in the form of a mixed solution with an organic solvent, but it may also be a mixed solution suitable for the reaction with amines in the following process. A method for isolating the polymer from the mixture is not specifically limited. Examples include isolation methods such as concentration, reprecipitation, etc. Specifically, for example, the polyimide compounds can be obtained by, after the termination of reaction, introducing a reaction mixture to methanol, ethanol, isopropyl alcohol, acetone, acetonitrile, water, or mixture thereof, decant ting the precipitate, separating by filtration etc., then washing again with solvent and drying. As a result, a polyimide compound can be obtained.

A polyimide compound of the present invention can be produced by the above method.

A solution containing a polyimide compound of the present invention refers to a solution of which a polyimide compound of the invention is dissolved in the above described solvent.

The polyimide compound of the present invention is soluble in the above solvent. Therefore, the polyimide compound itself can be suitably used as a raw material of polyamino acid polymer derived from a polyimide compound as well as a solution containing a polyimide compound dissolved in the above solvent.

Herein, polyamino acid polymer is produced from the polyimide compound of the present invention. A preparation process is not particular limited, and examples include the following preparation process. Firstly, polyimide compound is dissolved in a suitable solvent. The solvent is not particularly limited as long as it dissolves a polyimide compound and side reaction is not occurred with a reaction with amines. For example, N,N-dimethylformamide, dimethylsulfoxide, sulfolane, etc. can be used.

In these solvents, amines such as hexylamine, octylamine, decylamine, dodecylamine, tetradecylamine, octadecylamine, N,N-dimethylaminopropylamine, N,N-diethylaminopropylamine, N,N-dimethylaminoethylamine, aminomethanol, 2-aminoethanol, 3-aminopropanol, 4-aminobutanol, 5-aminopenthanol, 6-aminohexanol, methoxymethylamine, methoxyethylamine, methoxypropylamine, aminoethoxyethanol and the like are reacted with imide ring of polyimide compound. If amins are reacted with imide rings of polyimide compounds in the ratio of one mole of amine to one mole of imide ring, then the imide rings are opened by addition of the amins and grafted structure is formed in the side chain.

Continuously, an obtained reaction mixture is discharged in a poor solvent. This poor solvent is not specifically limited as long as it is a solvent in which the polyamino acid polymer is precipitated, and the solvent is not presented in a crystal of polyamino acid polymer after the polyamino acid polymer is filtered and dried. For example, acetonitrile, acetone, methanol, ethanol, etc., are used. The precipitates obtained by the above description is filtered and gathered, washed with poor solvent, and dried to obtain desired polyamino acid polymer.

The polyamino acid polymer derived from the polyimide compound of the present invention can be used by itself or the polymer solution in suitable solvent can be contained in cosmetics or an external preparations.

The cosmetics include a general cosmetics, specifically toilet water, moisturizing liquid, face washing oil, cleansing oil, cleansing milk, cleansing lotion, massage oil, moisture cream, shaving oil, shaving lotion, shaving mousse, sunscreen lotion, oil for sunburn, body lotion, body shampoo, hair shampoo, hair rinse, hair treatment, hair tonic oil, hair oil, hair mousse, perfumed oil, hair liquid, setting lotion, hair spray, hair bleacher, color rinse, color spray, permanent wave liquid, hand lotion, etc.

Further, the external preparation include an agent which can be included in a medicine for external use, and examples include a poultice, an analgesic, a coolant, an antipyretic, an agent for keeping warm, a disinfectant and germicide, a keratin softner, antifungal agent, sunscreening agent, skin breaching agent, a skin colorant, a granulation generator, epidermic former, a hair restorer, a deodorant, an antiperspirant, vitamin drop, etc.

The present invention will be described in details with reference to the following Examples. However, the present invention is by no means limited to these Examples. Further, weight average molecular weight (Mw) of the polymer is determined by GPC (gel permeation chromatography) on the basis of a polystyrene standard.

### [Analytical conditions for GPC]

Column: ShodexKD806M + KD-G
Developing solvent: N,N-dimethylformamide(0.01M LiBr)
Column Temperature: 45°C
Flow rate: 0.7 ml/min, Detection: RI
Sample Concentration: 0.25wt%, Sample Quantity: 100µl

Further, removing of water from the reaction system was carried out by following steps. Firstly, a mixed gas generated from polymerization system was condensed in a cooling tube. Secondly, an aqueous phase was separated and removed from liquid obtained above. Thereafter, only an organic phase was returned to the reaction mixture.

The description for a polyimide compound of the present invention is described below.
The state that the polyimide compound is dissolved in an aprotic organic solvent refers to homogeneous state in the liquid, and the solution can pass filter paper manufactured by Kiriyama glass works Co. (No. 5 A).

### EXAMPLE 1

As a reaction vessel, a separable flask equipped with a stirrer, heater, thermometer, dehydration apparatus (dean-stark trap), reflux apparatus (Dimroth) and a nitrogen line was used. During the reaction, the reaction was carried out while reaction system was sufficiently stirred. To the vessel, 66.6g of L-aspartic acid, 0.73g of lysine, 133.2g of sulfolane, 46g of xylene, and 26 g of 35% hydrochloric acid were charged. Then under the nitrogen atmosphere, azeotropic dehydration polymerization was carried out at 160 to 170°C for 10 hours. After the reaction, 133 g of N,N-dimethylformamide was charged to dilute. Continuously, the product was precipitated by pouring the reaction mixture to 1500ml of methanol. The slurry product was suction filtered, and recovered precipitate was dried with hot air dryer for overnight. The obtained polymer was homogeneously dissolved in N,N-dimethylformamide, and filtered through the filter paper manufactured by Kiriyama glass works Co. (No. 5A). The total solution passed through the filter paper. The weight average molecular weight was 513000.

### EXAMPLE 2

The same procedures as described in Example 1 were carried out except that 66.6g of L-aspartic acid, 0.93g of L-ornithine, 133.2g of sulfolane, 46g of xylene, and 26 g of 35% hydrochloric acid were charged. The obtained polymer was homogeneously dissolved in N,N-dimethylformamide. The solution was filtered and the total solution passed through. The weight average molecular weight was 279000.

### EXAMPLE 3

The same procedures as described in Example 1 were carried out except that 66.6g of L-aspartic acid, 0.66g of 1,6-hexanediamine, 133.2 g of sulfolane, 46g of xylene, and 26 g of 35% hydrochloric acid were charged. The obtained polymer was homogeneously dissolved in N,N-dimethylformamide. The solution was filtered and the total solution passed through. The weight average molecular weight was 411000.

### EXAMPLE 4

The same procedures as described in Example 1 were carried out except that 66.6g of L-aspartic acid, 0.73g of L-lysine, 133.2g of sulfolane, 46g of xylene, 19.2g of 85% phosphoric acid, and 19.6g of water were charged. The obtained polymer was homogeneously dissolved in N,N-dimethylformamide. The solution was filtered and the total solution passed through. The weight average molecular weight was 128000.

### [COMPARATIVE EXAMPLE 1]

The same procedures as described in Example 1 were carried out except that 66.6g of L-aspartic acid, 133.2g of sulfolane, 46g of xylene, and 26g of 35% hydrochloric acid were charged. The obtained polymer was homogeneously dissolved in N,N-dimethylformamide. The solution was filtered and the total solution passed through. The weight average molecular weight was 71000. A polymer having high molecular weight was not obtained.

### [COMPARATIVE EXAMPLE 2]

The same procedures as described in Example 1 were carried out except that 66.6g of L-aspartic acid, 0.73g of L-lysin, 133.2g of sulfolane, 46g of xylene, 16.4 g of titanyl(IV)acetylacetonato were charged. The obtained polymer was homogeneously dissolved in N,N-dimethylformamide. The solution was filtered and the total solution was passed through. The weight average molecular weight was 10000. A polymer having high molecular weight was not obtained.

### [COMPARATIVE EXAMPLE 3]

The same procedures as described in Example 1 were carried out except that 66.6g of L-aspartic acid, 5.8g of 1,6-hexanediamine, 133.2g of sulfolane, 46g of xylene, and 26 g of 35% hydrochloric acid were charged. The obtained polymer became gel in the reaction medium and therefore it became heterogeneous in N,N-dimethylformamide. The solution could not be filtrated. Since it did not pass through a filter, weight average molecular could not be determined.

### [COMPARATIVE EXAMPLE 4]

The same procedures as described in Example 1 were carried out except that 66.6g of L-aspartic acid, 9.1g of L-lysin, 133.2g of sulfolane, 46g of xylene, and 26 g of 35% hydrochloric acid were charged. The obtained polymer became gel in the reaction medium and therefore it became heterogeneous in N,N-dimethylformamide. The solution could not be filtrated. Since it did not pass through a filter, weight average molecular could not be determined.

### INDUSTRIAL APPLICABILITY

The polyimide compound having high molecula weight obtained by the preparation process of the present invention is useful as a raw material of polyamino acid derivative which has excellent coating-forming ability or thickening property. Further, according to the process of the present invention, a reaction time of polymerization can be reduced. In this regard, the productivity is increased, and industrially advantageous process can be provided.

A polyimide compound of the present invention and a polyamino acid polymer derived from a solution containing the compound can be suitably used as a formulation component of cosmetics or external preparations.
More specifically, cosmetics include toilet water, moisturizing liquid, face washing oil, cleansing oil, cleansing milk, cleansing lotion, massage oil, moisture cream, shaving oil, shaving lotion, shaving mousse, sunscreen lotion, oil for sunburn, body lotion, body shampoo, hair shampoo, hair rinse, hair treatment, hair tonic oil, hair oil, hair mousse, perfumed oil, hair liquid, setting lotion, hair spray, hair bleacher, color rinse, color spray, permanent wave liquid, hand lotion, etc. Further, as for an external preparation, it can all include to formulate an external medical use such as a poultice, an analgesic, a coolant, an antipyretic, an agent for keeping warm, a disinfectant and a germicide, a keratin softner, antifungal agent, sunburn inhibiting agent, skin breaching agent, a skin colorant, a granulation generator, epidermic former, a hair restorer, a deodorant, an antiperspirant, vitamin drop. Further it can be used to formulate with them.

## Claims

1. A polyimide compound crosslinked with polyamine which is soluble in the solvent containing aprotic polar organic solvent.

2. The polyimide compound according to claim 1, wherein said polyimide compound is soluble in said solvent containing the aprotic polar organic solvent in a concentration of 5% by mass or more at 25°C.

3. The polyimide compound according to claims 1 or 2, wherein said polyimide compound is succinimide polymer.

4. The polyimide compound according to any one of claims 1 to 3, wherein said aprotic polar organic solvent is a solvent containing N,N-dimethylformadmide and/or sulfolane.

5. The polyimide compound according to any one of claims 1 to 4, wherein said polyamine is lysine or ornithine.

6. The polyimide compound according to claim 1, wherein said polyimide compound is obtained by dehydration and condensation of amino acid or salt thereof in the presence of polyamine and protonic acid in the solvent containing aprotic polar organic solvent.

7. The polyimide compound according to claim 6, wherein said aprotic polar organic solvent is a solvent containing N,N-dimethylformamide and/or sulfolane.

8. The polyimide compound according to claim 6 or claim 7, wherein said protonic acid is hydrochloric acid.

9. The polyimide compound according to any one of the claims 6 to 8, wherein said polyimide compound is succinimide polymer.

10. The polyimide compound according to any one of claims 6 to 9, **characterized in that** 0.05 to 10 mol% of said polyamine is used with respect to the amount of said amino acid or salt thereof.

11. The polyimide compound according to any one of claims 6 to 10, wherein said polyamine is a lysine or an ornithine.

12. A solution containing a polyimide compound according to any one of claims 1 to 11.

13. A cosmetic containing a polyamino acid polymer derived from polyimide compound according to any one of claims 1 to 11.

14. An external preparation containing a polyamino acid polymer derived from the polyimide compound according to any one of claims 1 to 11.
